# EUROPÄISCHE PATENTANMELDUNG

(11) **EP 4 176 891 A1**
(43) Veröffentlichungstag der Anmeldung: **10.05.2023**
(21) Anmeldenummer: 22205554.3
(22) Anmeldetag: 04.11.2022
(51) Int. Cl.: A61K 36/185, A61K 36/424, A61K 36/53, A61K 36/9068, A61P 29/00

(54) **KRÄUTERMISCHUNG**

(30) Priorität: 05.11.2021 AT 508792021
(71) Anmelder: Rauh, Hans Jochen, 8020 Graz (AT)
(72) Erfinder: Rauh, Hans Jochen, 8020 Graz (AT)
(74) Vertreter: Schwarz & Partner Patentanwälte GmbH

(57) **Zusammenfassung**

Die vorliegende Erfindung betrifft eine Zusammensetzung, insbesondere eine Kräutermischung, umfassend getrocknete Pflanzen und/oder Pflanzenteile von *Gynostemma pentaphyllum,* von *Origanum vulgare* und von *Origanum majorana* und/oder *Thymus vulgaris* und/oder Extrakte dieser Pflanzen und/oder Pflanzenteile.

## Beschreibung

### TECHNISCHES GEBIET

Die vorliegende Erfindung betrifft Zusammensetzungen umfassend getrocknete Pflanzen oder Pflanzenteile oder Extrakte derselbigen.

### HINTERGRUND DER ERFINDUNG

Schmerzen, vor allem chronische Schmerzen, sind europa- und weltweit einer der häufigsten Ursachen für Krankenstände, Berufsunfähigkeit und Frühpensionierungen. In Europa, beispielsweise, wird die Anzahl der Menschen, die an chronischen Schmerzen leiden, auf über 100 Millionen geschätzt. Vor allem Gelenkschmerzen, Rückenschmerzen, Migräne und Rheuma belasten die betroffenen Patienten oft über viele Jahre. Daher können chronische Schmerzen als Volkskrankheit angesehen werden, die neben dem persönlichen Leid einen enormen volkswirtschaftlichen Schaden anrichtet.

Chronische Schmerzen finden sich in diversen Krankheitsbildern wieder. Daher stellt die Diagnose dieser Krankheiten aufgrund der vielen möglichen Ursachen für Ärzte eine große Herausforderung dar, so dass oftmals ein langer Zeitraum bis zur geeigneten Therapie vergeht. Insbesondere bei unspezifischen und chronischen Schmerzen stellen Schmerzmittel das Mittel der Wahl dar, um diese zumindest temporär zu lindern und den betroffenen Menschen Lebensqualität zu geben.

Viele Menschen stehen Schmerzmitteln skeptisch gegenüber. Einerseits weisen Schmerzmittel verschiedenste Nebenwirkungen wie Schleimhautentzündungen, Geschwüre oder Blutungen im Magen-Darm-Trakt auf. Andererseits sind am Markt zahlreiche Schmerzmittel wie Opioide erhältlich, die nachweislich zu Suchtverhalten führen können.

Eine sehr verbreitete Ursache für Schmerzen sind Entzündungen unterschiedlichster Art. Als problematisch werden insbesondere stille Entzündungen angesehen, da diese in aller Regel unentdeckt bleiben und eine mitunter große Belastung des Körpers darstellen. Stille Entzündungen sind in den allermeisten Fällen chronische Entzündungen, da diese in den meisten Fällen das Ergebnis von Übergewicht und Adipositas, Stress, falscher Ernährung, zu wenig Obst und Gemüse, zu vielen Transfette sowie mangelnder Bewegung sind. Auch die Lunge von Rauchern kann nach längerem Zigarettenkonsum Entzündungen auslösen, welche sich auf alle Organe übertragen.

Stille Entzündungen schwächen das Immunsystem und können die Entstehung von Erkrankungen wie Diabetes, Krebs, Herzinfarkt, Schlaganfall, Demenz, psychischen Krankheiten, COPD, Allergien, nicht-alkoholischer Fettleber, chronischen Schmerzen und Arteriosklerose begünstigen.

Es ist daher eine Aufgabe der vorliegenden Erfindung Zusammensetzungen zur Verfügung zu stellen, die in der Lage sind Schmerzen und/oder Entzündungen zu mildern oder die geeignet sind, die Nebenwirkungen von Schmerzmittel und Entzündungshemmern zu reduzieren bzw. deren Wirksamkeit zu unterstützen.

### ZUSAMMENFASSUNG DER ERFINDUNG

Die vorliegende Erfindung betrifft daher eine Zusammensetzung, insbesondere eine Kräutermischung oder ein Nahrungsergänzungsmittelzusammensetzung, umfassend getrocknete Pflanzen und/oder Pflanzenteile von *Gynostemma pentaphyllum,* von *Origanum vulgare* und von *Origanum majorana* und/oder *Thymus vulgaris* und/oder Extrakte dieser Pflanzen und/oder Pflanzenteile.

Es hat sich überraschender Weise ergeben, dass eine Zusammensetzung, welche getrocknete Pflanzen und/oder Pflanzenteile und/oder Extrakte dieser Pflanzen und/oder Pflanzenteile von *Gynostemma pentaphyllum* und von *Origanum vulgare* und von *Origanum majorana* und/oder *Thymus vulgaris* umfassen, entzündungshemmende und schmerzlindernd wirken. Die Anwesenheit von *Gynostemma pentaphyllum* und von *Origanum vulgare* ist für eine ausreichende Wirksamkeit jedenfalls erforderlich. Zusätzlich umfassen die erfindungsgemäßen entzündungshemmenden und schmerzlindernde Zusammensetzungen *Origanum majorana* und/oder *Thymus vulgaris.*

Ein weiterer Aspekt der vorliegenden Erfindung betrifft eine erfindungsgemäße Zusammensetzung zur Verwendung als Nahrungsergänzungsmittel oder Medikament.

Ein noch weiterer Aspekt der vorliegenden Erfindung betrifft eine erfindungsgemäße Zusammensetzung zur Verwendung bei der Behandlung von Schmerzen und/oder Entzündungen.

### BESCHREIBUNG DER AUSFÜHRUNGSFORMEN

Die Pflanzen bzw. Pflanzenteile, die zur Herstellung der erfindungsgemäßen Zusammensetzung verwendet werden, werden vorzugsweise in getrockneter Form vermengt und in einer entsprechenden Darreichungsform formuliert. Alternativ dazu können auch Extrakte dieser Pflanzen bzw. Pflanzenteile verwendet werden.

"Getrocknet", wie hier verwendet, bedeutet, dass die Pflanzen bzw. Pflanzenteile einen Wassergehalt von weniger als 20 Gew%, vorzugsweise von weniger als 18 Gew%, noch mehr bevorzugt von weniger als 15 Gew%, auf.

Ein "Extrakt" bzw. "Pflanzenextrakt", wie hierin verwendet, umfasst die Summe aller Stoffe verstanden, die durch Extraktion, bevorzugt durch Extraktion mit Kohlendioxid (z.B. mit überkritischem Kohlendioxid), Wasser und/oder Alkohol (z.B. Ethanol), aus dem pflanzlichen Rohmaterial herausgelöst werden. Je nach Darreichungsform (z.B. Kapsel) werden die Lösungsmittel anschließend entfernt, um einen trockenen Extrakt mit vorzugsweise weniger als 10 Gew%, noch mehr bevorzugt weniger als 5 Gew%, Lösungsmittel (wie z.B. Wasser) zu erhalten. Entsprechende Verfahren zur Extraktion von Pflanzeninhaltsstoffen sind dem Fachmann hinreichend bekannt.

Besonders bevorzugt umfasst die erfindungsgemäße Zusammensetzung Pflanzenteile, wobei besonders bevorzugte Pflanzenteile Blätter oder Blüten sind.

Gemäß einer bevorzugten Ausführungsform der vorliegenden Erfindung umfasst die erfindungsgemäße Zusammensetzung
- 15 bis 25 Gew%, vorzugsweise 18 bis 22 Gew%, getrocknete Pflanzen und/oder Pflanzenteile von *Gynostemma pentaphyllum* und/oder Extrakte dieser Pflanzen und/oder Pflanzenteile,
- 15 bis 25 Gew%, vorzugsweise 18 bis 22 Gew%, der getrockneten Pflanzen und/oder Pflanzenteile von *Origanum vulgare* und/oder Extrakte dieser Pflanzen und/oder Pflanzenteile und
- 15 bis 25 Gew%, vorzugsweise 18 bis 22 Gew%, der getrockneten Pflanzen und/oder Pflanzenteile von Origanum majorana und/oder *Thymus vulgaris* und/oder Extrakte dieser Pflanzen und/oder Pflanzenteile.

Die Konzentrationsangabe "Gew%" (Gewichtsprozent) bezieht sich auf die gesamte Zusammensetzung, welche neben den getrockneten Pflanzen bzw. Pflanzenteilen auch weitere Stoffe umfassen kann.

Das Verhältnis, d.h. Gewichtsverhältnis, von *Gynostemma pentaphyllum* und *Origanum vulgare* in der erfindungsgemäßen Zusammensetzung beträgt vorzugsweise 1,1:1 bis 1,1:1, am bevorzugtesten ca. 1:1. Auch das Verhältnis von *Gynostemma pentaphyllum* zu *Origanum majorana* und/oder *Thymus vulgaris* beträgt vorzugsweise 1,1:1 bis 1,1:1, am bevorzugtesten ca. 1:1.

In einer besonders bevorzugten Ausführungsform der vorliegenden Erfindung umfasst die erfindungsgemäße Zusammensetzung sämtliche getrocknete Pflanzen und/oder Pflanzenteile und/oder Extrakte dieser Pflanzen und/oder Pflanzenteile, die verschiedensten Ursprungs sind, in einem Verhältnis, d.h. Gewichtsverhältnis, von 1,1:1 bis 1,1:1, am bevorzugtesten ca. 1:1.

Gemäß einer weiteren bevorzugten Ausführungsform der vorliegenden Erfindung umfasst die Zusammensetzung Kieselerde, vorzugsweise 15 bis 25 Gew%, noch mehr bevorzugt 18 bis 22 Gew%, Kieselerde.

Kieselerde wird der erfindungsgemäßen Zusammensetzung zugegebenen, um den Anteil an Mineralien in der Zusammensetzung zu erhöhen. Es hat sich gezeigt, dass Kieselerde, insbesondere die darin enthaltenen Mineralien, in der erfindungsgemäßen Zusammensetzung unterschiedliche Körperteile und Organe, insbesondere Knochen, Gelenke, Haut und Haare, stärken und unterstützen kann.

Gemäß einer noch weiteren bevorzugten Ausführungsform der vorliegenden Erfindung umfasst die Zusammensetzung trockene Pflanzenteile von *Zingiber officinale* und/oder *Cannabis sativa* und/oder Extrakte dieser Pflanzenteile, vorzugsweise in einer Konzentration von 15 bis 25 Gew%.

Die erfindungsgemäße Zusammensetzung kann neben den oben genannten Pflanzen bzw. Pflanzenteile auch trockene Pflanzenteile von *Zingiber officinale* und/oder *Cannabis sativa* und/oder Extrakte dieser Pflanzenteile umfassen. Bevorzugte Pflanzenteile von *Zingiber officinale* und *Cannabis sativa* sind Wurzeln/Wurzelknolle bzw. Blüten.

Gemäß einer bevorzugten Ausführungsform der vorliegenden Erfindung sind die getrockneten Pflanzen und/oder Pflanzenteile gemahlen.

Die getrockneten Pflanzen bzw. Pflanzenteile werden vorzugsweise durch Mahlen zerkleinert. Die zerkleinerten Pflanzenteile weisen vorzugsweise eine Partikelgröße von 0,5 µm bis 5 mm, noch mehr bevorzugt von 1 µm bis 4 mm, gemessen an den in den Pflanzenpartikeln am weitest entfernten Punkten, auf.

Gemäß einer weiteren bevorzugten Ausführungsform der vorliegenden Erfindung liegt die Zusammensetzung in einer oral zu verabreichenden Kapsel, vorzugsweise in einer magensaftresistenten Kapsel, vor, wobei die Kapsel vorzugsweise Gelatine oder ein veganes Kapselmaterial umfasst. Als veganes bzw. pflanzliches Kapselmaterial wird vorzugsweise modifizierte oder nicht-modifizierte Zellulose oder Stärke (z.B. Stärke aus Kartoffeln und/oder Mais) eingesetzt. Besonders bevorzugt ist die Verwendung von Gelatine oder Hydroxypropylmethylzellulose (HPMC). Je nach Größe der Kapsel umfasst diese 100 bis 500 mg, vorzugsweise 120 bis 400 mg, noch mehr bevorzugt 150 bis 300 mg, der erfindungsgemäßen Zusammensetzung.

Ein weiterer Aspekt der vorliegenden Erfindung betrifft eine erfindungsgemäße Zusammensetzung zur Verwendung als Nahrungsergänzungsmittel oder Medikament.

Ein noch weiterer Aspekt der vorliegenden Erfindung betrifft eine erfindungsgemäße Zusammensetzung zur Verwendung bei der Behandlung von Schmerzen und/oder Entzündungen.

Es hat sich überraschender Weise gezeigt, dass die erfindungsgemäße Zusammensetzung vorteilhafte Wirkungen in Bezug auf die Behandlung und Linderung von Schmerzen und Entzündungen in Säugetieren und Menschen zeigt.

Als Nahrungsergänzungsmittel kann die erfindungsgemäße Zusammensetzung ebenfalls zur Linderung von Schmerzen, vor allem chronischen Schmerzen, und Entzündungen, insbesondere stillen Entzündungen, beitragen bzw. die Wirkung anderer Medikamente unterstützen, wodurch die Dosis dieser Medikamente signifikant reduziert werden kann.

Die erfindungsgemäße Zusammensetzung wird vorzugsweise oral verabreicht.

Gemäß einer bevorzugten Ausführungsform der vorliegenden Erfindung wir die erfindungsgemäße Zusammensetzung einem Säugetier, vorzugsweise einem Rind, einem Hund, einer Katze, einem Huhn, einem Schaf, einer Ziege, einem Hamster oder einem Menschen verabreicht wird.

Gemäß einer bevorzugten Ausführungsform der vorliegenden Erfindung beträgt die Dosis, mit der die erfindungsgemäße Zusammensetzung einem Säugetier oder Menschen, vorzugsweise täglich, verabreicht wird, vorzugsweise zwischen 1 und 30 Milligramm, noch mehr bevorzugt zwischen 2 und 25 Milligramm, noch mehr bevorzugt zwischen 5 und 20 Milligramm, noch mehr bevorzugt zwischen 8 und 15 Milligramm, insbesondere ca. 10 Milligramm, pro Kilogramm Körpergewicht. Diese Dosis ist ausreichend, um einen positiven Effekt bei der Behandlung von Schmerzen oder Entzündungen, beispielsweise, zu erzielen. Je nach Größe der Kapsel bzw. je nach der Menge an der erfindungsgemäßen Zusammensetzung in der Kapsel kann eine unterschiedliche Anzahl von Kapseln verabreicht werden. Beispielsweise besteht bei einem Körpergewicht von 100 Kilogramm eine Tagesdosis aus 6 Kapseln, vorzugsweise 4 Kapseln, mit einem Füllgewicht von 250 Milligramm pro Kapsel.

Die vorliegende Erfindung wird anhand des folgenden Beispiels eingehender erläutert.

### BEISPIEL

### Studienaufbau

Neun Patienten (Alter zwischen 25 und 47 Jahren) mit chronischen Schmerzen im Rücken- und/oder Nackenbereich und stillen nicht lokalisierbaren Entzündungen im Körper erhielten über einen Zeitraum von sechs Monaten unterschiedliche Zusammensetzungen oral in Kapselform verabreicht. Die Patienten wurden über den Inhalt der Kapseln nicht im Detail informiert. Es wurde den Patienten nur mitgeteilt, dass sich in den Zusammensetzungen unterschiedliche Kräuter befinden, die schmerzlindernde Wirkung aufweisen.

Die Patienten nahmen mit einem Glas Wasser täglich vier Kapseln (zwei am Morgen, zwei am Abend) zu sich. Die Patienten protokollierten die Schmerzen zwei Mal täglich (am Morgen und am Abend) auf einer Skala von 1 (kein Schmerz) bis 10 (größter vorstellbarer Schmerz). Zusätzlich wurde den Patienten am Beginn der Studie und anschließend einmal monatlich Blut abgenommen, um den Entzündungsfaktor CRP (C-Reaktives Protein) zu messen zu bestimmen.

### Zusammensetzungen

Folgende Zusammensetzungen wurden den Patienten verabreicht:
Zusammensetzung A (Placebo):
   - 150 mg Kieselerde
Zusammensetzung B:
   - 30 mg getrocknete und gemahlene Blätter von *Gynostemma pentaphyllum*
   - 120 mg Kieselerde
Zusammensetzung C:
   - 30 mg getrocknete und gemahlene Blätter von *Gynostemma pentaphyllum*
   - 30 mg getrocknete und gemahlene Blätter von *Origanum vulgare*
   - 90 mg Kieselerde
Zusammensetzung D:
   - 30 mg getrocknete und gemahlene Blätter von *Gynostemma pentaphyllum*
   - 30 mg getrocknete und gemahlene Blätter von *Origanum vulgare*
   - 30 mg getrocknete und gemahlene Blätter von *Origanum majorana*
   - 60 mg Kieselerde
Zusammensetzung E:
   - 30 mg getrocknete und gemahlene Blätter von *Gynostemma pentaphyllum*
   - 30 mg getrocknete und gemahlene Blätter von *Origanum vulgare*
   - 30 mg getrocknete und gemahlene Blätter von *Thymus vulgaris*
   - 60 mg Kieselerde
Zusammensetzung F:
   - 30 mg getrocknete und gemahlene Blätter von *Gynostemma pentaphyllum*
   - 30 mg getrocknete und gemahlene Blätter von *Origanum vulgare*
   - 30 mg getrocknete und gemahlene Blätter von *Thymus vulgaris*
   - 30 mg getrocknete und gemahlene Blätter von *Origanum majorana*
   - 30 mg Kieselerde

### Studienprotokoll

Zu Beginn der Studie wurde der CRP Wert als Ausgangspunkt im Blut aller Patienten bestimmt. Den Patienten wurde 4 Wochen lang Zusammensetzung A als Placebo verabreicht. Im Anschluss an diese Eingangsphase nahmen jeweils drei Patienten je 8 Wochen lang Zusammensetzung A (Patienten Nr. 1 bis 3), Zusammensetzung B (Patienten Nr. 4 bis 6) bzw. Zusammensetzung C (Patienten Nr. 7 bis 9). Ab der 13. Woche nahmen alle Patienten für 4 Wochen Zusammensetzung A zu sich. Ab der 17. Woche nahmen jeweils drei Patienten je 8 Wochen lang Zusammensetzung D (Patienten Nr. 1 bis 3), Zusammensetzung E (Patienten Nr. 4 bis 6) bzw. Zusammensetzung F (Patienten Nr. 7 bis 9) ein.

### Ergebnisse

Die Patienten führten ein Schmerztagebuch, in dem sie zwei Mal täglich die Intensität ihrer Schmerzen vermerkten (Skala 1 bis 10).

Die Durchschnittswerte (arithmetisch gerundet) der Schmerzskala der einzelnen Patienten in den entsprechenden Behandlungswochen sind in der folgenden Tabelle angegeben:

| **Patient Nr./Woche** | **1-4** | **5/6** | **7/8** | **9/10** | **11/12** | **13-16** | **17/18** | **19/20** | **21/22** | **23/24** |
|---|---|---|---|---|---|---|---|---|---|---|
| 1 | 4 | 4 | 5 | 5 | 4 | 4 | 3 | 3 | 2 | 3 |
| 2 | 5 | 4 | 7 | 6 | 6 | 5 | 4 | 4 | 3 | 3 |
| 3 | 6 | 6 | 5 | 6 | 6 | 5 | 6 | 5 | 4 | 4 |
| 4 | 4 | 4 | 3 | 3 | 4 | 3 | 3 | 2 | 3 | 3 |
| 5 | 4 | 4 | 4 | 4 | 5 | 4 | 4 | 3 | 3 | 3 |
| 6 | 8 | 6 | 7 | 6 | 8 | 8 | 6 | 5 | 6 | 6 |
| 7 | 7 | 8 | 8 | 6 | 8 | 7 | 6 | 4 | 2 | 2 |
| 8 | 5 | 6 | 6 | 5 | 5 | 5 | 4 | 3 | 3 | 3 |
| 9 | 4 | 3 | 3 | 4 | 5 | 3 | 4 | 2 | 3 | 1 |

Die Ergebnisse belegen eindeutig, dass die Schmerzen mit Zusammensetzung F am meisten gesenkt werden konnten (siehe Patienten 7 bis 9). Mit den Zusammensetzungen D und E konnte ebenfalls eine Reduktion der Schmerzen beobachtet werden, die jedoch geringer als mit Zusammensetzung F ist.

Die CRP-Werte im Blut der Patienten wurden ca. alle vier Wochen erhoben:

| **Patient Nr./Woche** | **0** | **4** | **8** | **12** | **16** | **20** | **24** |
|---|---|---|---|---|---|---|---|
| 1 | <5mg/l | <5mg/l | <5mg/l | <5mg/l | <5mg/l | <5mg/l | <5mg/l |
| 2 | 28mg/l | 35mg/l | 16mg/l | 25mg/l | 30mg/l | 18mg/l | 15mg/l |
| 3 | 45mg/l | 50mg/l | 38mg/l | 54mg/l | 40mg/l | 32mg/l | 25mg/l |
| 4 | 12mg/l | <5mg/l | 8mg/l | <5mg/l | <5mg/l | <5mg/l | <5mg/l |
| 5 | 18mg/l | 26mg/l | 14mg/l | 23mg/l | 24mg/l | 13mg/l | 6mg/l |
| 6 | 90mg/l | 78mg/l | 85mg/l | 75mg/l | 60mg/l | 55mg/l | 26mg/l |
| 7 | 56mg/l | 60mg/l | 44mg/l | 50mg/l | 53mg/l | 38mg/l | 32mg/l |
| 8 | 43mg/l | 38mg/l | 49mg/l | 53mg/l | 44mg/l | 15mg/l | 8mg/l |
| 9 | 58mg/l | 75mg/l | 46mg/l | 54mg/l | 48mg/l | 24mg/l | <5mg/l |

Die Messungen der CRP Werte ergab, dass dieser Wert bei Patienten, denen Zusammensetzung F verabreicht wurde, über die Behandlungsdauer signifikant reduziert werden konnte. Zusammensetzungen A bis C zeigten hingegen keinen und Zusammensetzungen D und E einen CRP-reduzierenden Effekt.

## Patentansprüche

1. Zusammensetzung, insbesondere Kräutermischung, umfassend getrocknete Pflanzen und/oder Pflanzenteile von *Gynostemma pentaphyllum,* von *Origanum vulgare* und von *Origanum majorana* und/oder *Thymus vulgaris* und/oder Extrakte dieser Pflanzen und/oder Pflanzenteile.

2. Zusammensetzung gemäß Anspruch 1, **dadurch gekennzeichnet, dass** die Zusammensetzung
- 15 bis 25 Gew% getrocknete Pflanzen und/oder Pflanzenteile von *Gynostemma pentaphyllum* und/oder Extrakte dieser Pflanzen und/oder Pflanzenteile,
- 15 bis 25 Gew% der getrockneten Pflanzen und/oder Pflanzenteile von *Origanum vulgare* und/oder Extrakte dieser Pflanzen und/oder Pflanzenteile und
- 15 bis 25 Gew% der getrockneten Pflanzen und/oder Pflanzenteile von *Origanum majorana* und/oder *Thymus vulgaris* und/oder Extrakte dieser Pflanzen und/oder Pflanzenteile
umfasst.

3. Zusammensetzung gemäß Anspruch 1 oder 2, **dadurch gekennzeichnet, dass** die Zusammensetzung Kieselerde, vorzugsweise 15 bis 25 Gew% Kieselerde, umfasst.

4. Zusammensetzung gemäß einem der Ansprüche 1 bis 3, **dadurch gekennzeichnet, dass** die Zusammensetzung trockene Pflanzenteile von *Zingiber officinale* und/oder *Cannabis sativa* und/oder Extrakte dieser Pflanzenteile, vorzugsweise in einer Konzentration von 15 bis 25 Gew%, umfasst.

5. Zusammensetzung gemäß einem der Ansprüche 1 bis 4, **dadurch gekennzeichnet, dass** die getrockneten Pflanzen und/oder Pflanzenteile gemahlen sind.

6. Zusammensetzung gemäß einem der Ansprüche 1 bis 5, **dadurch gekennzeichnet, dass** die Zusammensetzung in einer oral zu verabreichenden Kapsel, vorzugsweise in einer
magensaftresistenten Kapsel, vorliegt, wobei die Kapsel vorzugsweise Gelatine oder ein veganes Kapselmaterial umfasst.

7. Zusammensetzung gemäß einem der Ansprüche 1 bis 6 zur Verwendung als Nahrungsergänzungsmittel oder Medikament.

8. Zusammensetzung gemäß einem der Ansprüche 1 bis 6 zur Verwendung bei der Behandlung von Schmerzen und/oder Entzündungen.

9. Zusammensetzung zur Verwendung gemäß Anspruch 8, **dadurch gekennzeichnet, dass** die Zusammensetzung einem Säugetier, vorzugsweise einem Rind, einem Hund, einer Katze, einem Huhn, einem Schaf, einer Ziege, einem Hamster oder einem Menschen verabreicht wird.
